# EUROPEAN PATENT SPECIFICATION

(11) **EP 4 554 465 B1**
(45) Date of publication and mention of the grant of the patent: **07.10.2026**
(21) Application number: 23751155.5
(22) Date of filing: 10.07.2023
(51) Int. Cl.: A61B 5/022, A61B 5/00

(54) **PRESSURE CUFF OVERTIGHTENING DETECTION ALGORITHM**
ALGORITHMUS ZUR ERKENNUNG VON DRUCKMANSCHETTENÜBERSPANNUNG
ALGORITHME DE DÉTECTION DE SERRAGE EXCESSIF DE BRASSARD DE PRESSION

(30) Priority: 14.07.2022 US 202263389338 P
(43) Date of publication of application: 21.05.2025
(73) Proprietor: Becton, Dickinson and Company, Franklin Lakes, NJ 07417-1880 (US)
(72) Inventor: MORSY, Yasser, Khaled, Bridgeville, PA 15017 (US); JIAN, Zhongping, Irvine, CA 92614 (US); REUDERINK, Boris, 3818 BG Amersfoort (NL); KUIJKENS, Hans, Jean Paul, 1101 BE Amsterdam (NL); VAN GOUDOEVER, Jeroen, 1101 BE Amsterdam (NL); MA, George, Hua, Irvine, CA 92614 (US)
(74) Representative: Venner, Julia Ann
(86) International application number: PCT/US2023/027280
(87) International publication number: WO 2024/015305

(56) References cited:
- US-A1- 2019 167 195
- US-A1- 2021 259 629

## Description

### CROSS-REFERENCE TO RELATED APPLICATION(S)

This application claims the benefit of U.S. Provisional Application No. 63/389,338, filed July 14, 2022, and entitled "PRESSURE CUFF OVERTIGHTENING DETECTION ALGORITHM,".

### BACKGROUND

The present disclosure relates generally to arterial pressure monitoring, and more specifically to evaluating fit and/or location quality of hemodynamic sensor through analysis of the arterial pressure signal.

Volume clamping is a technique for non-invasively measuring arterial pressure in which pressure is applied to a patient's finger in such a manner that arterial pressure may be balanced by a time varying pressure to maintain a constant arterial volume. In a properly fitted and calibrated system, the applied time varying pressure is equal to the arterial pressure in the finger. The applied time varying pressure may be measured to provide a reading of the patient's arterial pressure.

This may be accomplished by a finger cuff that is arranged around a finger of a patient. The finger cuff may include an infrared light source, an infrared sensor, and an inflatable bladder. The infrared light may be sent through the finger in which a finger artery is present. The infrared sensor picks up the infrared light and the amount of infrared light registered by the sensor may be inversely proportional to the artery diameter and indicative of the pressure in the artery.

In the finger cuff implementation, by inflating the bladder in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure. By controlling the pressure of the inflatable bladder such that the diameter of the finger artery is kept constant, the blood pressure may be monitored in very precise detail as the pressure in the inflatable bladder is directly linked to the blood pressure. In a typical present day finger cuff implementation, a volume clamp system is used with the finger cuff. The volume clamp system typically includes a pressure generating system and a regulating system that includes: a pump, a valve, and a pressure sensor in a closed loop feedback system that are used in the measurement of the arterial volume. To accurately measure blood pressure, the feedback loop provides sufficient pressure generating and releasing capabilities to match the pressure oscillations of the subject's blood pressure.

Current finger cuffs are easy to place on a patient's finger. However, it is important that the finger cuff is placed on the patient's finger correctly in order for the blood pressure measurement system to obtain and report correct blood pressure measurement values. In order to obtain a correct attachment of the finger cuff to the finger, the finger cuff needs to be placed on the finger at the correct depth, correct angle, and with the correct tightness. Current finger cuffs are often made from a flexible material that a healthcare provider wraps around the patient's finger and locks into place by simple attachment mechanisms, such as, Velcro. These current types of finger cuffs require that the healthcare provider is able to control all three of these variables (e.g., correct depth, correct angle, and correct tightness), simultaneously, while attaching the finger cuff. Unfortunately, this may result in erroneous placement and tightness of the finger cuff. This erroneous placement and tightness may result in an unsuccessful reading of the patient's blood pressure. Even where the healthcare work initially places and tightens the finger cuff correctly, the finger cuff can become incorrectly placed or tightened by patient movement or a change in the patient's condition. For example, the patient's extremities may develop edema. Swelling as a result of edema or other cause may also result in an unsuccessful reading of the patient's blood pressure.

US 2021/0259629 A1 relates to a system for monitoring arterial pressure of a patient. The system determines a score that is predictive of responsiveness of the patient to a therapy. Sensed hemodynamic data representative of an arterial pressure waveform of the patient are received by a hemodynamic monitor. Magnitude data and trend data are derived from the hemodynamic data. The score that is predictive of the responsiveness of the patient to the therapy is determined based on the magnitude data and the trend data of the hemodynamic parameter. A representation of the score is output.

### SUMMARY

A system for monitoring the fit of a hemodynamic sensor (e.g., a finger cuff) to a patient, in accordance with an exemplary example of this disclosure, includes a hardware unit. The hardware unit includes a hardware processor, an analog-to-digital converter (ADC), and a system memory. The system further includes a signal distortion detection code and weighting module stored in the system memory and a sensory alarm. The hardware processor is configured to execute the signal distortion detection code to obtain hemodynamic data, which may be converted by the ADC, from a signal received from the hemodynamic sensor on an ongoing basis. The code is further configured to obtain an arterial pressure waveform based on the hemodynamic data and extract a plurality of features from the first arterial pressure waveform indicative of distortion of the first arterial pressure waveform associated with a fit or location quality of hemodynamic sensor to the patient. Using the weighting module and the extracted features (i.e., distortion parameters), the code is further configured to determine a distortion score and invoke a sensor alarm if the distortion score satisfies a predetermined criterion.

### BRIEF DESCRIPTION OF THE DRAWINGS

FIG. 1 is a schematic of an exemplary patient monitoring system.
FIG. 2 is a schematic of an exemplary patient monitoring system that includes a finger cuff.
FIG. 3A is a schematic of a finger cuff and a pressure controller of an exemplary hemodynamic sensor.
FIG. 3B is a schematic of a finger cuff in an uninstalled state.
FIG. 4A, FIG. 4B, and FIG. 4C are exemplary charts comparing arterial pressure parameters of a finger cuff with a normal fit to arterial pressure parameters of a finger cuff with an overtightened fit.
FIG. 5 is a schematic depicting features of an arterial pressure waveform taken a patient's finger along with associated brachial and radial arterial pressure waveforms derived from the arterial pressure waveform.
FIG. 6 is a chart depicting exemplary features of the radial arterial pressure waveform.
FIG. 7 is a chart depicting exemplary features of the brachial arterial pressure waveform.
FIG. 8 is a flow chart illustrating steps of the signal distortion detection method.

### DETAILED DESCRIPTION

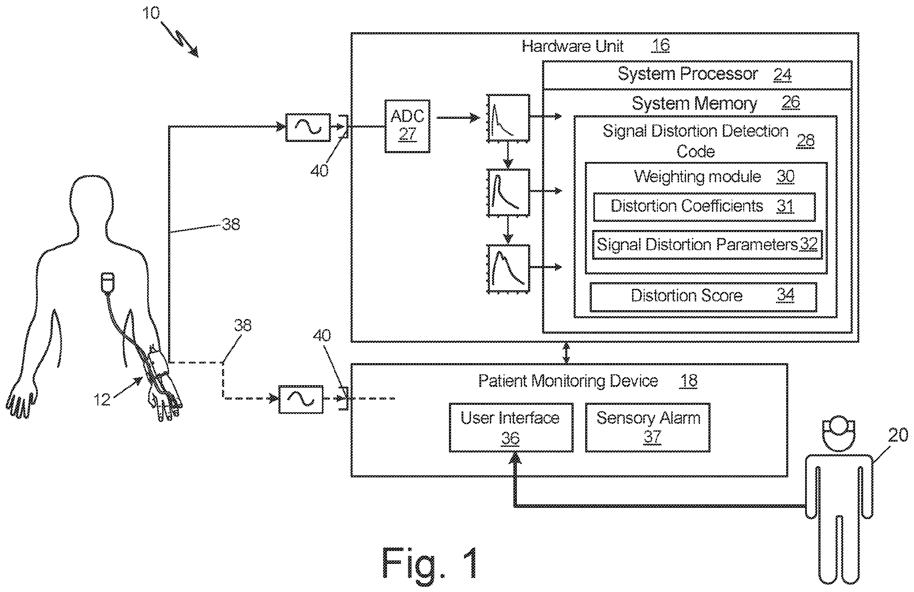

FIG. 1 is a schematic depicting components of an exemplary patient monitoring system capable of evaluating the fit and/or placement of sensors attached to a patient. As shown, patient monitoring system 10 includes hemodynamic sensor 12 fit to patient 14, hardware unit 16, and patient monitoring device 18.

Hemodynamic sensor 12 can be a non-invasive sensor fitted about an appendage of patient 14 and, in some instances, at a specific location along the patient's appendage. Misfitting or mislocating hemodynamic sensor 12 may occur incidentally during application by practitioner 20 or may develop from patient movement and/or changes in the patient's condition (e.g., swelling of the patient's appendage). When misfitted and/or mislocated, hemodynamic sensor 12 may provide erroneous hemodynamic data to hardware unit 16, impairing monitoring of patient 14.

Hardware unit 16 includes system processor 24, system memory 26, and analog-to-digital (ADC) converter 27, which interface with patient monitoring device 18. In some instances, hardware unit 16 is discrete from patient monitoring device 18, which may be installable into a corresponding receptacle or slot of patient monitoring device 18. In other examples, hardware unit 16 integrates with internal components of patient monitoring device 18. In further examples, one or more components and/or described functionality can be distributed among multiple hardware units 16, patient monitoring devices 18, and/or other patient monitoring equipment.

System processor 24 executes signal distortion detection code 28, which uses weighting module 30 and signal distortion parameters 32 to determine distortion score 34 representing fit quality and/or position quality of hemodynamic sensor 12. Examples of system processor 24 can include any one or more of a microprocessor, a controller, a digital signal processor (DSP), an application specific integrated circuit (ASIC), a field-programmable gate array (FPGA), or other equivalent discrete or integrated logic circuitry.

System memory 26 can be configured to store information within hardware unit 16 during operation. As illustrated in FIG. 1, system memory 26 stores signal distortion detection code 28. System memory 26, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 26 can include volatile and non-volatile computer-readable memories. Examples of volatile memories can include random access memories (RAM), dynamic random-access memories (DRAM), static random-access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Hardware unit 16 receives hemodynamic data from hemodynamic sensor 12. Hemodynamic data can include any one or more features of an arterial pressure waveform or derived from an arterial pressure waveform. The arterial pressure waveform can be measured at a radial location, a brachial location, or a finger location of patient 14. Further, features from multiple waveforms (e.g., from one or more arterial waveforms taken at a radial location, a brachial location, and/or a finger location) can be used in combination by signal distortion detection code 28. For example, a non-exhaustive list of potential arterial pressure waveform features includes mean arterial pressure, maximum arterial pressure, minimum arterial pressure, pulse rate, pulse pressure, maximum and/or minimum rate of pressure change with respect to time during a heartbeat (or a subset of heartbeats), maximum and/or minimum rate of pressure change with respect of time during the systolic phase and/or diastolic phase of a heartbeat (or a subset of heartbeats), end diastolic pressure, diastolic gradient, systolic pressure gradient, pulse transit time, cardiac output, stroke volume, blood temperature, systolic rise area, diastolic average pressure, systemic vascular resistance, exponential constant associated with systolic decay, among other possible parameters.

Weighting module 30 applies distortion coefficients 31, determined via training, to signal distortion parameters 32, which are features extracted from an arterial pressure waveform. Certain signal distortion parameters 32 may be indicative of hemodynamic sensor 12 with a proper fit and/or proper location while other signal distortion parameters 32 may be indicative of hemodynamic sensor 12 with an improper fit and/or improper location. Based on distortion coefficients 31 applied by weighting module 30 and signal distortion parameters 32, signal distortion detection code 28 determines distortion score 34 representative of the fit quality and/or location quality of hemodynamic sensor 12.

The selection of distortion coefficients 31 and/or signal distortion parameters 32 can be accomplished via training (e.g., offline training) of the distortion detection model using machine learning or other techniques to minimize a cost function representing the error of the distortion detection model output to the true value of training subsets that define properly fit and improper fit hemodynamic sensors 12. Training of the distortion detection model can be accomplished using a variety of supervised and/or unsupervised techniques known in the art. One exemplary technique includes obtaining arterial pressure feature datasets from hemodynamic sensors 12 known to have a proper fit and proper location and from different hemodynamic sensors 12 known to have an improper fit and/or improper location. Based on features associated with two or more datasets, logistic regression can be used to identify which features are indicative of proper fit and/or proper location of hemodynamic sensors 12 and which features are indicative of improper fit and/or improper location of hemodynamic sensors 12. Using this feature subset, further training of the distortion model determines weighting factors (i.e., distortion coefficients 31) for weighting module 30. The subset of features extracted from the arterial pressure waveform and indicative of a properly fit and/or improperly fit hemodynamic sensor 12 become distortion signal parameters 32.

Distortion score 34 can be a normalized value between 0 and 1 (or between 0 and 100, or other normalized ranges) with, in some examples, a higher value representing a higher likelihood that hemodynamic sensor 12 is misfit or mispositioned and a lower value representing a lower likelihood that hemodynamic sensor 12 is misfit or mispositioned. In another example, the normalized range of the distortion scores can be subdivided into two or more continuous and sequential subranges, each subrange indicative of the fit quality and/or location quality of hemodynamic sensor 12 to patient 14. The first of the two subranges (e.g., a distortion score value between 0 and 50 within a normalized range from 0 to 100) can be indicative of a properly fit hemodynamic sensor 12. The second subrange (e.g., a distortion score between 51 and 100 within a normalized range from 0 to 100) can indicate improperly fit or mislocated hemodynamic sensor 12. When the normalized range consists of two subranges, system 10 can provide a binary indication of a properly fit/location or improperly fit/located hemodynamic sensor 12. In other examples, the normalized range of distortion scores 34 can be subdivided into at least three continuous and sequential subranges. The first of the three subranges (e.g., a distortion score value between 0 and 30 within a normalized range from 0 to 100) can be indicative of a properly fit hemodynamic sensor 12. The second subrange (e.g., a distortion score between 31 and 60 within a normalized range from 0 to 100) can indicate a potential improper fit and/or potential mislocated hemodynamic sensor 12. The third subrange (e.g., a distortion score between 61 and 100 within a normalized range from 0 to 100) can indicate improperly fit or mislocated hemodynamic sensor 12. In this way, the second subrange (i.e., intermediate subrange) may provide an indication that hemodynamic sensor 12 should be refit or repositioned whereas the third subrange may indicate that hemodynamic sensor 12 should be replaced.

One example equation for determining distortion score 34 is as follows: $Distortion Score = \frac{100}{1 + {e}^{- \left({w}_{0}+Σ{w}_{i} {x}_{i}\right)}}$ Equation 1
for i = 1, 2, 3, ..., 13 (i.e., a total number of distortion parameters in the distortion detection model model)
where, xᵢ's are the example parameters determined from hemodynamic data measured by hemodynamic sensor 12, wᵢ's are the corresponding parameter weights (i.e., coefficients), and w₀ is a bias term.
x₁ = mean arterial pressure
x₂ = maximum arterial pressure
x₃ = minimum arterial pressure
x₄ = pulse rate
x₅ = pulse pressure
x₆ = maximum rate of pressure change with respect to time during a heartbeat (or a subset of heartbeats),
x₇ = minimum rate of pressure change with respect to time during a heartbeat (or a subset of heartbeats)
x₈ = maximum rate of pressure change with respect of time during the systolic phase of a heartbeat (or a subset of heartbeats)
x₉ = minimum rate of pressure change with respect of time during the systolic phase of a heartbeat (or a subset of heartbeats)
x₁₀ = maximum rate of pressure change with respect of time during the diastolic phase of a heartbeat (or a subset of heartbeats)
x₁₁ = minimum rate of pressure change with respect of time during the diastolic phase of a heartbeat (or a subset of heartbeats)
x₁₂ = end diastolic pressure
x₁₃ = diastolic gradient
x₁₄ = systolic pressure gradient
x₁₅ = pulse transit time
x₁₆ = cardiac output
x₁₇ = stroke volume
x₁₃ = blood temperature
x₁₉ = systolic rise area
x₂₀ = diastolic average pressure
x₂₁ = systemic vascular resistance
x₂₂ = exponential constant associated with systolic decay

Distortion score 34 can be determined using one, a selected number, or all of distortion parameters 32, and some examples of determining distortion score 34 can include the use of other parameters from multiple arterial pressure waveforms associated with different measurement locations (e.g., a radial location, a brachial location, and/or a finger location).

Patient monitoring device 18 may be any type of medical electronic device that may read, collect, process, display, etc., physiological readings/data of a patient including blood pressure, as well as any other suitable physiological patient readings. Patient monitoring device 18 can include user interface 36 and sensory alarm 37. In some examples, power/data cable 38 may connect to hardware unit 16 via one or more input and/or output connectors 40 as shown with a solid line in FIG. 1. In other examples, patient monitoring device 18 can be connected to hemodynamic sensor 12 via power/data cable 38 and one or more input and/or output connectors 40 as indicated by a dashed line in FIG. 1. In this example, hardware unit 16 receives hemodynamic data via patient monitoring device 18.

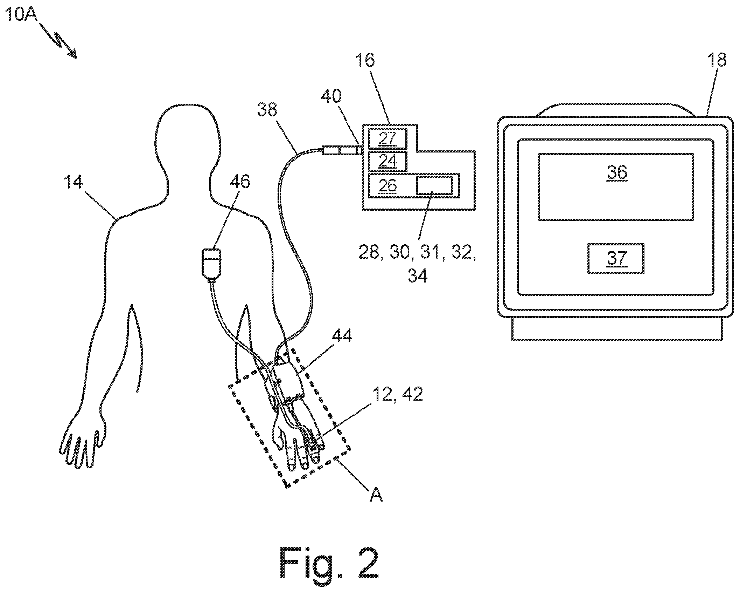

User interface 36 can be a liquid crystal display (LCD), a light-emitting diode (LED) display, an organic light-emitting diode (OLED) display, or other display device suitable for providing information to users in graphical form. In some examples, such as the example of FIG. 2, user interface 36 can be a touch-sensitive and/or presence-sensitive display device configured to receive user input in the form of gestures, such as touch gestures, scroll gestures, zoom gestures, swipe gestures, or other gesture input.

Power/data cable 38 may transmit data to and from patient monitoring device 18 and may provide power from the patient monitoring device 18 to hemodynamic sensor 12 and/or other components associated with the operation of hemodynamic sensor 12. In some examples, patient monitoring device 18 can be a hemodynamic monitor. Input and/or output (I/O) connectors 40 are configured for wired connection (e.g., electrical and/or communicative connection) with one or more peripheral components, such as one or more hemodynamic sensors

In operation, hardware unit 16 receives hemodynamic data from patient 14 via hemodynamic sensor 12 that can be digitally converted by analog-to-digital converter (ADC) 27. In response to receiving hemodynamic data of patient 14, hardware unit 16 executes signal distortion detection code 28 to determine distortion score 34 representing the fit quality and/or location quality of hemodynamic sensor 12 to patient 14. Based on distortion score 34, signal distortion detection code 28 causes patient monitoring device 18 to provide an indication of fit quality and/or location quality via user interface 36 and/or sensory alarm 37. The indication of fit quality and/or location quality of hemodynamic sensor 12 can include displaying distortion score 34 directly via user interface 36. In other examples, the indication of fit quality and/or location quality may include displaying a message or warning representative of the fit quality and/or location quality of hemodynamic sensor 12. Additionally, hardware unit 16 can invoke sensory alarm 37, such as an audible alarm, a haptic alarm, or other sensory alarm in response to determining that distortion score satisfies predetermined criteria or criterion (e.g., a distortion score within one of the predefined subranges). Accordingly, hardware unit 16 can provide a warning or message to patient monitoring device 18 so that practitioner 20 can correct improper fit and/or improper position of hemodynamic sensor 12 and/or replace hemodynamic sensor 12.

FIG. 2 is a schematic of exemplary arterial pressure monitoring system 10A. Arterial pressure monitoring system 10A is an exemplary implementation of patient monitoring system 10 shown in FIG. 1 and, except for noted differences, includes the components of patient monitoring system 10. Accordingly, arterial pressure monitoring system 10A includes hardware unit 16 and patient monitoring device 18. As shown, hardware unit 16 is a discrete module that can be inserted into a slot of patient monitoring device 18. Hardware unit 16 includes system processor 24 for executing signal distortion detection code 28 and system memory 26 for storing signal distortion detection code 28 and weighting module 30 for determining distortion score 34 based on distortion coefficients 31 and distortion parameters 32. Hardware unit additionally includes analog-to-digital (ADC) converter 27. Patient monitoring device 18 includes user interface 36 and sensory alarm 37.

Hemodynamic sensor 12 takes the form of finger cuff 42, pressure controller 44, and heart reference sensor 46. In one example, finger cuff 42 may be attached to the finger (or toe) of patient 14, and pressure controller 44 can be attached to the body of patient 14 with an attachment bracelet that wraps around the patient's wrist or hand. It should be appreciated that this is just one example of attaching pressure controller 44 and that any suitable way of attaching pressure controller 44 to a patient's body or in close proximity to a patient's body may be utilized.

Pressure controller 44 can be further connected to hardware unit 16 via power/data cable 38, which connects to hardware unit 16 via input/output connector 40. Finger cuff 42 provides hemodynamic data to hardware unit 16 representative of arterial pressure of arteries within a finger (or toe) of patient 14. Heart reference sensor 46 connects to pressure controller 44 to provide a signal representative of differential pressure between the patient's finger and heart elevation. This differential pressure signal enables pressure controller 44 to compensate hemodynamic data for changes in arterial pressure due to the patient's finger having a different elevation than the patient's heart. Together, finger cuff 42, pressure controller 44, and heart reference sensor 46 provide arterial waveform of patient 14 to hardware unit 16.

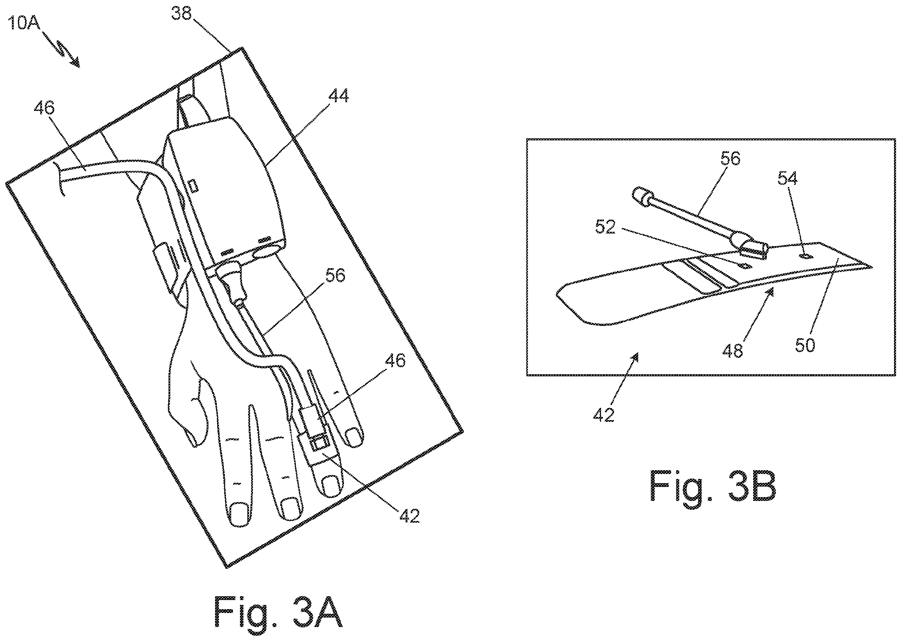

FIG. 3A is a schematic of finger cuff 42 and pressure controller 44 of exemplary hemodynamic sensor 12, which is an enlarged view of region A in FIG. 2. FIG. 3B depicts finger cuff 42 in an uninstalled state to illustrate features of finger cuff 42 hidden when fit to patient 14.

As shown in FIG. 3B, finger cuff 42 includes substrate 48, inflatable bladder 50, light-emitting diode (LED) 52, photodiode (PD) 54, and tube 56. Substrate 48 is a flexible or semi-rigid material that forms an exterior layer of finger cuff 42. Inflatable bladder 50 mounts to the interior surface of substrate 48 that is fluidly connected to tube 56. LED 52 is spaced from PD 54 along a longitudinal direction of substrate 48 and each of LED 52 and PD 54 is mounted to an interior surface of substrate 48.

Referring to FIG. 3A, pressure controller 44 can include a small internal pump, a small control valve, a pressure sensor, and control circuitry. The pump can be disposed along an internal conduit connecting tube 56 of finger cuff 42 to an intake port of pressure controller 44 that communicates with an ambient environment. The control circuitry can be configured to control the pneumatic pressure applied by the internal pump to the bladder of the finger cuff 42 to replicate the patient's blood pressure based upon measuring the signal received from the LED-PD pair of finger cuff 42. One end of heart reference sensor 46 attaches to the patient adjacent finger cuff 42 as shown in FIG. 3A while an opposite end of heart reference sensor 46 attaches to the patient's body at heart-level as shown in FIG. 2. Further, the control circuitry may be configured to control the opening of the control valve to release pneumatic pressure from the bladder. Alternatively, the control valve can be replaced with an orifice that is not controlled. In these examples, bladder pressure within finger cuff 42 varies based on operational speed or periodic operation of the internal pump and/or position of the control valve.

Continuing with this example, a patient's hand may be placed beside the patient's body in a sitting or a prone position for measuring a patient's blood pressure with the blood pressure measurement system 10. Pressure controller 44 of system 10A may be coupled to bladder 50 of the finger cuff 42 in order to provide pneumatic pressure to bladder 50 for use in blood pressure measurement. Pressure controller 44 may be coupled to hardware unit 16 (shown in FIGS. 1-2) through power/data cable 38. Accordingly, pressure controller 44 operates to vary an internal pressure of bladder 50 according to the volume clamp method, previously described, and outputs a signal representative of the arterial pressure within a finger (or toe) of patient 14 as a function of time (i.e., an arterial pressure waveform).

The arterial pressure waveform, which initially represents the arterial pressure within the patient's finger, can be mathematically transformed to represent arterial pressure waveforms at location such as a brachial site and/or a radial site of patient 14. As used herein, such radial arterial pressure waveforms and brachial arterial pressure waveforms are reconstructed from the finger arterial pressure waveform rather than measured directly (e.g., via an invasive catheterization technique).

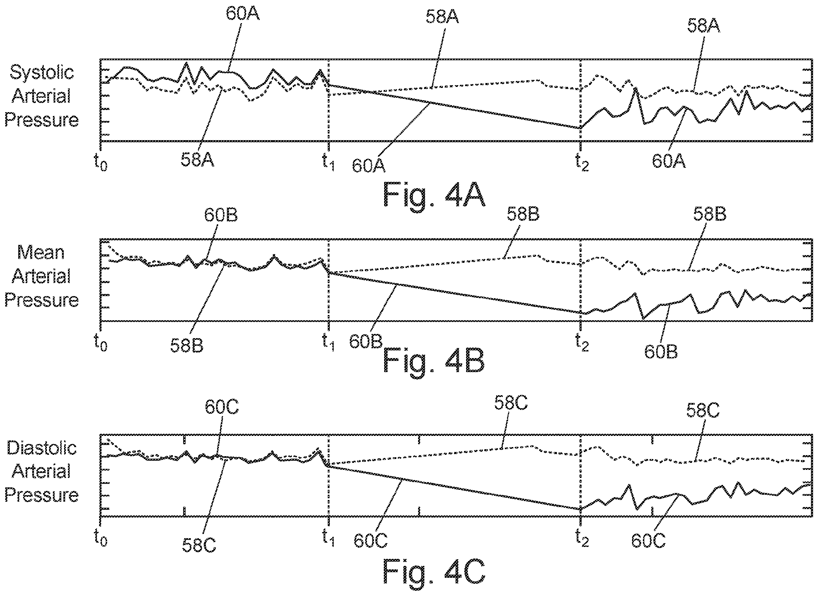

FIG. 4A, FIG. 4B, and FIG. 4C are charts depicting exemplary arterial pressure features representative of a radial site within patient 14 and measured using finger cuff 42, pressure controller 44, and heart reference sensor 46 (shown in FIG. 2). As shown in each of FIG. 4A, FIG. 4B, and FIG. 4C, an arterial pressure feature from a properly fit finger cuff 42 and from an improperly fit finger cuff 42 are compared. Based on this comparison, arterial pressure features with the tightest correlation to a properly fit and/or improperly fit finger cuff 42 can be identified and used to determine distortion score 34 via Equation 1. Repeating this process for each potential arterial pressure waveform feature may identify a set of arterial pressure waveform features indicative of a properly fit finger cuff 42 and/or an improperly fit finger cuff 42.

Referring to FIG. 4A, 4B, and 4C, arterial parameters 58A, 58B, and 58C are indicative of systolic arterial pressure, mean arterial pressure, and diastolic arterial pressure, respectively, of a properly fitted and located first finger cuff. Arterial pressure parameters 60A, 60B, and 60C are representative of systolic arterial pressure, mean arterial pressure, and diastolic arterial pressure, respectively, produced by a second finger cuff. Prior to time step t₁, both first and second finger cuffs have proper tightness. The measurement of the first finger cuff and the second finger cuff were both stopped at time step t₁, the second cuff was then intentionally overtightened, and the measurement continues at time t₂. The first finger cuff and the second finger cuff are each installed on separate fingers of the same patient 14 in order to compare arterial pressure output from each figure cuff. As shown in FIG. 4A, FIG. 4B, and FIG. 4C, overtightening the second finger cuff at time t₂ causes arterial parameters 60A, 60B, and 60C to deviate relative to a properly fit and located first finger cuff. In this particular example, arterial parameters 60A, 60B, and 60C decrease relative to corresponding parameters produced by a properly fit first finger cuff (e.g., arterial parameters 58A, 58B, and 58C). Local maximum pressures and/or local minimum pressures of parameters 60A, 60B, and 60C relative to mean values of respective parameters 60A, 60B, and 60C are greater than or less than corresponding local maximum pressures and local minimum pressures of respective parameters produced by the first finger cuff (e.g., arterial parameters 58A, 58B, and 58C). Accordingly, these deviations can be used to identify arterial pressure features indicative of a proper fit and/or improper fit of finger cuff 42. Once identified, the deviation of the arterial pressure features relative to a properly fit finger cuff 42 characterize the effects of improperly fitting finger cuff 42 to a patient's finger.

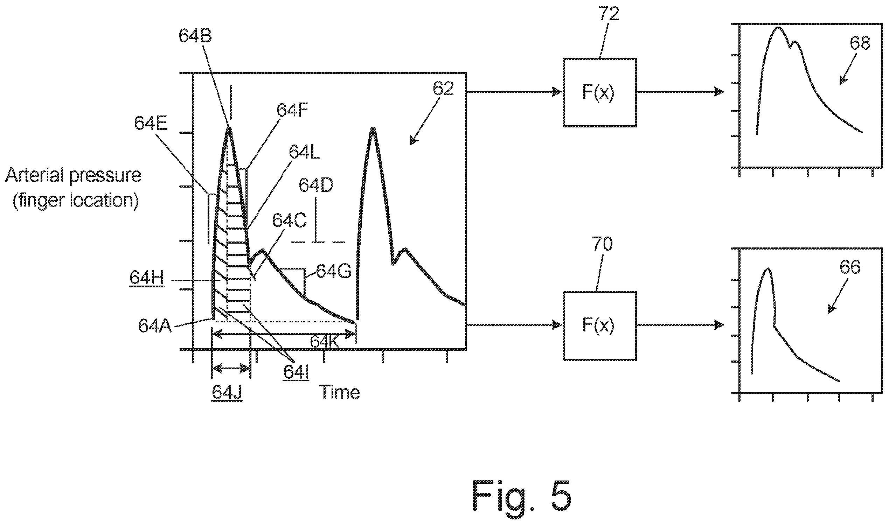

FIG. 5 is a schematic depicting features of exemplary arterial pressure waveform 62 taken at a patient's finger along with reconstructed brachial and radial arterial pressure waveforms derived from the arterial pressure waveform. Arterial pressure waveform 62 includes at least features 64A, 64B, 64C, 64D, 64E, 64F, 64G, 64H, 64I, 64J, 64K, and 64L, collectively arterial pressure features 64A-64L.

Features 64A, 64B, and 64C are the minimum arterial pressure (i.e., end diastolic pressure), maximum arterial pressure, and dichotic notch pressures, respectively, of a heartbeat. Feature 64D is the mean arterial pressure of a heartbeat (or series of heartbeats). Feature 64E is the maximum rate of pressure change during systolic rise. The maximum rate of pressure change during systolic decay is indicated by feature 64F. The maximum rate of change of the diastolic phase is represented by feature 64G. Feature 64H represents the area under arterial pressure waveform 62 between the minimum arterial pressure 64A and maximum arterial pressure 64B (i.e., during systolic rise) of a heartbeat, and feature 64I represents the area under arterial pressure waveform 62 between minimum arterial pressure 64A and dichotic pressure 64C (i.e., during the systolic phase). Feature 64J can be the time of the systolic phase for a given heartbeat. Feature 64K is the time between successive heartbeats of arterial pressure waveform 62 from which pulse rate can be determined. The exponential decay constant of the systolic phase is represented by feature 64L.

Additional features can be derived from arterial pressure features 64. For example, stroke volume is proportional to the systolic phase area (i.e., feature 64I) and, as such, can derived from systolic phase area (i.e., feature 64I). Cardiac output equals stroke volume multiplied by pulse rate (i.e., feature 64K). Systemic vascular resistance can be determined from the difference between mean arterial pressure and central venous pressure, divided by cardiac output.

Similar features can be extracted from radial arterial pressure waveform 66 and/or brachial arterial pressure waveform 68. Radial arterial pressure waveform 66 is derived from finger arterial pressure waveform 62 using transfer function 70. Brachial arterial pressure waveform 68 is derived from finger arterial pressure waveform 62 using transfer function 72. Each of transfer function 70 and transfer function 72 are determined by correlating arterial pressure features 64 of finger arterial pressure waveform 62 to corresponding features of radial arterial pressure waveform 66 and brachial arterial pressure waveform 68.

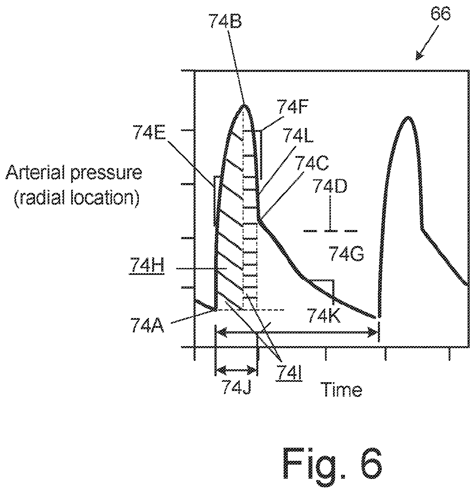

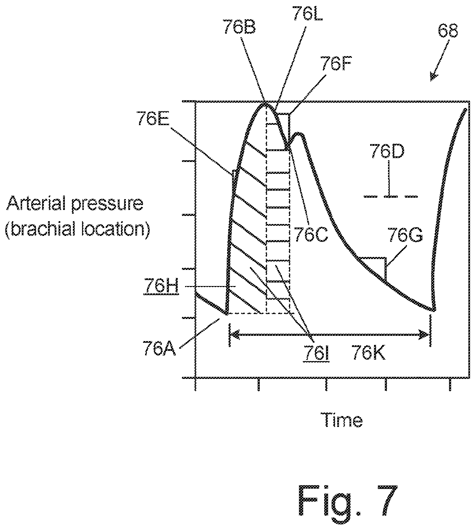

FIG. 6 is a chart depicting exemplary features 74A, 74B, 74C, 74D, 74E, 74F, 74G, 74H, 74I, 74K, and 74L, collectively radial arterial pressure features 74A-74L, of reconstructed radial pressure waveform 66. FIG. 7 is a chart depicting exemplary features 76A, 76B, 76C, 76D, 76E, 76F, 76G, 76H, 761, 76K, and 76L, collectively brachial arterial pressure features 76A-76L, of reconstructed brachial pressure waveform 68. Each of features 74A-74L and features 76A-76L correspond to like features 64A-64L of finger pressure waveform 62 shown in FIG. 5.

Accordingly, corresponding features 74A-74L are determined from reconstructed radial arterial pressure waveform 66. Features 74A, 74B, and 74C are the minimum arterial pressure (i.e., end diastolic pressure), maximum arterial pressure, and dichotic notch pressures, respectively, of a heartbeat from reconstructed radial arterial pressure waveform 66. Feature 74D is the mean arterial pressure of a heartbeat (or series of heartbeats). Feature 74E is a maximum rate of pressure change during systolic rise. The maximum rate of pressure change during systolic decay is indicated by feature 74F. The maximum rate of change of the diastolic phase is represented by feature 74G. Feature 74H represents the area under radial arterial pressure waveform 66 between the minimum arterial pressure 74A and maximum arterial pressure 74B (i.e., during systolic rise) of a heartbeat, and feature 74I represents the area under radial arterial pressure waveform 66 between minimum arterial pressure 74A and dichotic pressure 74C (i.e., during the systolic phase). Feature 74J can be the time of the systolic phase for a given heartbeat. Feature 74K is the time between sequential heartbeats of reconstructed radial arterial pressure waveform 66 from which pulse rate can be determined. The exponential decay constant of the systolic phase is represented by feature 74L. Each of features 74A-74L is determined from radial arterial pressure waveform 66, which is reconstructed or derived from finger arterial pressure waveform 62.

Likewise, corresponding features 76A-76L are determined from brachial arterial pressure waveform 68. Features 76A, 76B, and 76C are the minimum arterial pressure (i.e., end diastolic pressure), maximum arterial pressure, and dichotic notch pressures, respectively, of a heartbeat from brachial arterial pressure waveform 68. Feature 76D is the mean arterial pressure of a heartbeat (or series of heartbeats). Feature 76E is a maximum rate of pressure change during systolic rise. The maximum rate of pressure change during systolic decay is indicated by feature 76F. The maximum rate of change of the diastolic phase is represented by feature 76G. Feature 76H represents the area under brachial arterial pressure waveform 68 between the minimum arterial pressure 76A and maximum arterial pressure 76B (i.e., during systolic rise) of a heartbeat, and feature 76I represents the area under brachial arterial pressure waveform 68 between minimum arterial pressure 76A and dichotic pressure 76C (i.e., during the systolic phase). Feature 76J can be the time of the systolic phase for a given heartbeat. Feature 76K is the time between sequential heartbeats of brachial arterial pressure waveform 68 from which pulse rate can be determined. The exponential decay constant of the systolic phase is represented by feature 76L. Each of features 76A-76L is determined from brachial arterial pressure waveform 68, which is reconstructed or derived from finger arterial pressure waveform 62.

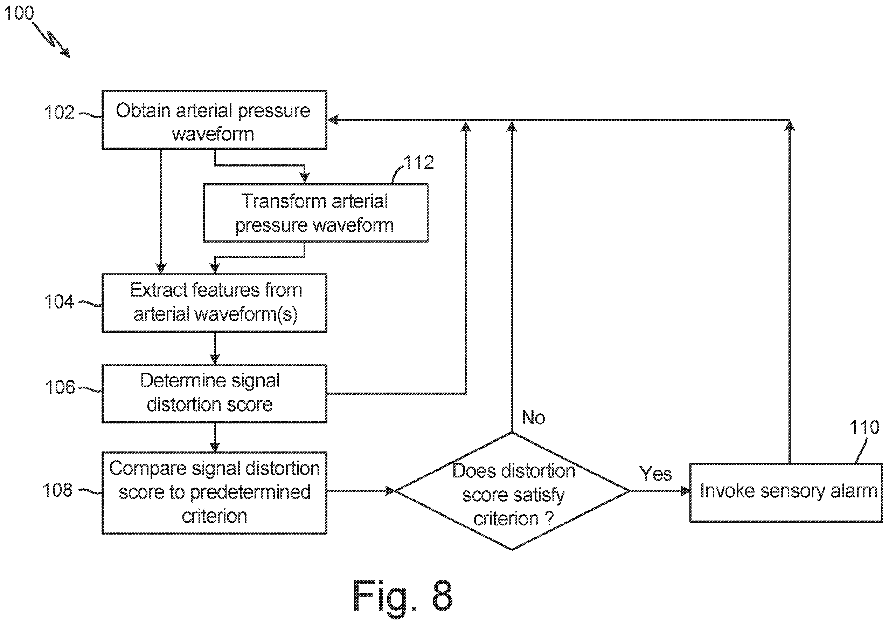

FIG. 8 is a flowchart describing signal distortion detection method 100 for evaluating the fit of finger cuff 42, or another hemodynamic sensor 12. Method 100 includes steps 102, 104, and 106. In further examples, method 100 can include one or more of steps 108, 110, and 112. The sequence depicted in FIG.8 is for illustrative purposes only and is not meant to limit method 100 in any way as it is understood that the portions of method 100 can proceed in a different logical order, additional or intervening portions can be included, or described portions of method 100 can be divided into multiple portions, or described portions of method 100 can be omitted without detracting from the described above.

In step 102, an arterial pressure waveform is obtained from a patient monitoring system. For example, pressure controller 44 may vary a bladder pressure of finger cuff 42 in accordance with the volume clamp to obtain hemodynamic data representative of the patient's arterial pressure waveform. This hemodynamic data may be converted into digital hemodynamic data prior by analog-to-digital converter 27 located within pressure controller 44, hardware unit 16, or patient monitoring device 18. Based on the hemodynamic data, hardware unit 16 continuously determines an arterial pressure waveform (i.e., arterial pressure as a function of time). As generally represented by patient monitoring system 10, a different hemodynamic sensor 12 could be used that produces hemodynamic data representative of a patient's arterial pressure. In either example, step 102 includes measuring or calculating an arterial pressure waveform based on measurements of a hemodynamic sensor sensitive to fit and/or placement on the patient.

Step 104 includes extracting one or more features from the arterial pressure waveform measured or determined in step 102. As discussed above, features of the arterial pressure waveform can include one or more of mean arterial pressure, maximum arterial pressure, minimum arterial pressure, pulse rate, pulse pressure, maximum and/or minimum rate of pressure change with respect to time during a heartbeat (or a subset of heartbeats), maximum and/or minimum rate of pressure change with respect of time during the systolic phase and/or diastolic phase of a heartbeat (or a subset of heartbeats), end diastolic pressure, diastolic gradient, systolic pressure gradient, pulse transit time, cardiac output, stroke volume, blood temperature, systolic rise area, diastolic average pressure, systemic vascular resistance, exponential constant associated with systolic decay, among other possible features. Any one or more of the features can be extracted from finger arterial pressure waveform 62 or, as discussed further below, radial arterial pressure waveform 66 or brachial arterial pressure waveform 68, which are reconstructed or derived from finger arterial pressure waveform 62. In other examples, features can be extracted from each of the finger, radial, and brachial waveforms, or any two of the arterial waveforms (e.g., two arterial waveforms selected from finger arterial pressure waveform 62, radial arterial pressure waveform 66, and brachial arterial pressure waveform 68).

Step 106 includes determining distortion score 34 based on one or more of the extracted features. For example, a subset of the potential features can be incorporated into a distortion detection model using training methods discussed above. Continuing with this example, distortion coefficient associated with each extracted feature can be used to determine distortion score 34 based on Equation 1. In one example, distortion score 34 is determined based on one or more of average diastolic pressure, stroke volume, systolic vascular resistance of radial arterial pressure waveform 66 and systolic rise area and exponential decay constant of the systolic phase of brachial arterial waveform 68. Steps 102, 104, and 106 or steps 102, 104, 106, and 112 can be repeated to determine a trend of distortion score 34 during a period of time. In some examples, method 100 determines distortion score 34 as an average distortion score 34 over a period of time (e.g., a twenty-second time interval). In other examples, method 100 may determine whether distortion score 34 is increase or decreasing. In such examples, method 100 may determine a rate of increase or a rate of decrease of distortion score 34.

In step 108, distortion score 34 or a trend of distortion scores 34 (e.g., an average distortion score 34, rate of increasing distortion score 34, a rate of decreasing distortion score 34, or a combination of these distortion score trend parameters) is compared to a predetermined criterion or set of criteria. For example, distortion score 34 can be compared to one of multiple subranges of a nominal total range of distortion score 34. In a further example, distortion score 34 can be compared to a value of distortion score 34 and a trend of distortion score over a period time, the value of distortion score 34 associated with one or more subranges of a nominal range of distortion scores 34. In a further example, distortion score 34 can be a value within a nominal range divided into two consecutive subranges (e.g., a first subrange between 0 and 50 and a second range between 51 and 100 for a nominal range in which distortion score 34 can be a value between 0 and 100). For distortion scores 34 within the first subrange, hardware unit 16 may cause one or more of the following actions to occur: a) display distortion score 34 on user interface 36 of patient monitoring device 18 and b) display an indication (e.g., a message or other status indication) that hemodynamic sensor 12 is properly fit to patient 14. Alternatively, no message or status indication is provided when distortion score 34 indicates a proper fit and location of hemodynamic sensor 12. In this instance, steps 102, 104, and 106 can be repeated to provide continuous or periodic monitoring of hemodynamic sensor 12. For distortion scores 34 within the second subrange, hardware unit 16 can invoke sensory alarm in step 110. Thereafter, steps 102, 104, 106, 108, and 110 can be repeated continuously invoking sensory alarm until medical practitioner refits, repositions, and/or replaces hemodynamic sensor 12.

In some examples of method 100, the arterial pressure waveform obtained in step 102 can be mathematically transformed in step 112 to represent an arterial pressure waveform at a location different than the location of hemodynamic sensor 12. For example, arterial pressure waveforms produced by finger cuff 42 are representative of the arterial pressure within the patient's finger (or toe). The finger arterial pressure waveform can be transformed into an arterial pressure waveform at a radial location of the patient and/or a brachial location of the patient. Features of brachial and/or radial arterial pressure waveforms can be extracted in step 104 and a distortion score 34 determined in step 106 based on one or more features extracted from one or more of the finger arterial pressure waveform, the radial arterial pressure waveform, and the brachial arterial pressure waveform. Thereafter, the distortion score determined in accordance with step 100 can be compared to a predetermined criterion or criteria in step 108. Based on this comparison, the sensory alarm can be invoked in step 110. Whether or not sensory alarm is invoked, steps 102, 104, 106, 108, 110, and 112 are repeated to provide continuous and/or periodic monitoring of hemodynamic sensor 12 fit and/or location.

### Discussion of Possible Examples

The following are non-exclusive descriptions of possible examples of the present invention.

### System for monitoring hemodynamic data of a patient

A system for monitoring hemodynamic data of a patient according to an example of this disclosure includes, among other possible things, a hardware unit, a signal distortion detection code, a hemodynamic sensor, and a sensory alarm. The hardware unit includes a hardware processor, an analog-to-digital converter (ADC), and a system memory. The signal distortion detection code is stored in the system memory and includes a weighting module. The hemodynamic sensor is coupled to the hardware unit and fitted about an appendage of the patient. The hardware processor is configured to execute the signal distortion detection code to evaluate the fit of the hemodynamic sensor to the patient.

The system of the preceding paragraph can optionally include, additionally and/or alternatively, any one or more of the following features, configurations and/or additional components.

A further example of the foregoing system, wherein the hardware processor can be configured to execute the signal distortion detection code to obtain digital hemodynamic data converted, by the ADC, from a signal received from the hemodynamic sensor on an ongoing basis.

A further example of any of the foregoing systems, wherein the hardware processor can be configured to execute the signal distortion detection code to obtain a first arterial pressure waveform based on the digital hemodynamic data.

A further example of any of the foregoing systems, wherein the hardware processor can be configured to execute the signal distortion detection code to extract a plurality of features from the first arterial pressure waveform.

A further example of any of the foregoing systems, wherein the plurality of features can be indicative of a fit of the hemodynamic sensor to the patient.

A further example of any of the foregoing systems can include determining, using the weighting module, a distortion score corresponding to the likelihood that the hemodynamic sensor is misfit to the patient based on a weighted combination of the plurality of features.

A further example of any of the foregoing systems can include invoking the sensory alarm if the distortion score satisfies a predetermined distortion criterion.

A further example of any of the foregoing systems can include a patient monitoring device.

A further example of any of the foregoing systems, wherein the hardware unit can be housed within the patient control unit.

A further example of any of the foregoing systems, wherein the hemodynamic sensor can be a finger cuff that includes an inflatable bladder configured to wrap around finger of the patient, a light-emitting diode, and a photodiode spaced along a longitudinal dimension of the inflatable bladder from the light-emitting diode.

A further example of any of the foregoing systems, wherein the first arterial pressure waveform can be representative of an arterial pressure within the finger of the patient.

A further example of any of the foregoing systems can include a pressure control unit coupled to the finger cuff by a tube to receive the signal.

A further example of any of the foregoing systems, wherein the signal can be indicative of an air pressure within the inflatable bladder of the finger cuff.

A further example of any of the foregoing systems, wherein the pressure control unit can include a control valve, and a pump disposed along a conduit connecting the tube of the finger cuff to an intake port of the pressure control unit.

A further example of any of the foregoing systems, wherein the intake port of the pressure control unit can communicate with an ambient environment.

A further example of any of the foregoing systems, wherein the control valve of the pressure control unit can operate to vary the air pressure within the inflatable bladder based on a blood pressure of the patient.

A further example of any of the foregoing systems, wherein obtaining the first arterial pressure waveform includes transforming the digital hemodynamic data such that the first arterial pressure waveform is representative of one of a radial arterial pressure waveform and brachial arterial pressure waveform.

A further example of any of the foregoing systems, wherein the plurality of features can include at least one of an average diastolic pressure, a stroke volume, and a systemic vascular resistance derived from the first arterial pressure waveform representative of the radial arterial pressure waveform.

A further example of any of the foregoing systems, wherein the plurality of features can include at least one of a systolic rise area and an exponential decay constant of the systolic phase derived from the brachial arterial waveform.

A further example of any of the foregoing systems, wherein the plurality of features can include each of an average diastolic pressure, a stroke volume, and a systemic vascular resistance derived from the first arterial pressure waveform representative of the radial arterial pressure waveform and each of a systolic rise area and an exponential decay constant of the systolic phase derived a second arterial pressure waveform representative of the brachial arterial waveform.

A further example of any of the foregoing systems, wherein the plurality of features can include at least one of mean arterial pressure, maximum arterial pressure, minimum arterial pressure, pulse rate, pulse pressure, maximum rate of pressure change with respect to time, minimum rate of pressure change with respect to time, end diastolic pressure, diastolic gradient, systolic pressure gradient, pulse transit time, cardiac output, stroke volume, blood temperature, systolic rise area, diastolic average pressure, systemic vascular resistance, and exponential constant associated with systolic decay.

A further example of any of the foregoing systems, wherein at least one feature of the plurality of features can be extracted from a finger arterial pressure waveform, a radial arterial pressure waveform, or a brachial arterial pressure waveform.

A further example of any of the foregoing systems, wherein the predetermined distortion criterion can be based on at least one of a value of the distortion score and a trend of the distortion score over a time interval.

A further example of any of the foregoing systems, wherein the distortion score can equal a value within a nominal range of distortion scores subdivided into at least two subranges.

A further example of any of the foregoing systems, wherein the predetermined distortion criterion can be based on a value of the distortion score associated with a threshold between subranges.

A further example of any of the foregoing systems, wherein extracting the plurality of features from the first arterial pressure waveform can include extracting the plurality of features from a time interval starting with the application of the hemodynamic sensor to the patient or a physiological change of the patient.

A further example of any of the foregoing systems, wherein the time interval can be greater than or equal to twenty seconds and less than or equal to sixty seconds.

### Method for use by a system for monitoring hemodynamic data of a patient

A method for use by any of the foregoing exemplary systems for monitoring hemodynamic data of the patient includes, among other possible steps, obtaining, by the signal distortion detection code executed by the hardware processor, digital hemodynamic data converted, by the ADC, from a signal received from the hemodynamic sensor on an ongoing basis. The method includes obtaining, by the signal distortion detection code executed by the hardware processor, a first arterial pressure waveform based on the digital hemodynamic data and extracting, by the signal distortion detection code executed by the hardware processor, a plurality of features from the first arterial pressure waveform. The plurality of features is indictive of distortion of the first arterial pressure waveform associated with a fit of the hemodynamic sensor to the patient. The method includes determining, by the signal distortion detection code using the weighting module and executed by the hardware processor, a distortion score corresponding to the likelihood that the hemodynamic sensor is misfit to the patient based on a weighted combination of the plurality of features. The method includes invoking, by the signal distortion detection code executed by the hardware processor, the sensory alarm if the distortion score satisfies a predetermined distortion criterion.

The method of the preceding paragraph can optionally include, additionally and/or alternatively, any one or more of the following features, configurations, additional components, and/or steps.

A further example of the foregoing method, wherein the predetermined distortion criterion can be based on at least one of a value of the distortion score and a trend of the distortion score over a time interval.

A further example of any of the foregoing methods, wherein obtaining the first arterial pressure waveform can include transforming, by the signal distortion detection code executed by the hardware processor, the digital hemodynamic data such that the first arterial pressure waveform can be representative of one of a radial arterial pressure waveform and a brachial arterial pressure waveform.

A further example of any of the foregoing methods, wherein the plurality of features can include at least one of an average diastolic pressure, a stroke volume, and a systemic vascular resistance, each feature derived from the first arterial pressure waveform representative of the radial arterial pressure waveform.

A further example of any of the foregoing methods can include obtaining, by the signal distortion detection code executed by the hardware processor, a second arterial pressure waveform based on the digital hemodynamic data.

A further example of any of the foregoing methods, wherein obtaining the second arterial pressure waveform can include transforming, by the signal distortion detection code executed by the hardware processor, the digital hemodynamic data such that the second arterial pressure waveform can be representative of the other of the radial arterial pressure waveform and the brachial arterial pressure waveform.

A further example of any of the foregoing methods, wherein the plurality of features can include at least one of a systolic rise area and an exponential decay constant of the systolic phase, each feature derived from the brachial arterial waveform.

A further example of any of the foregoing methods, wherein the plurality of features can include each of an average diastolic pressure, a stroke volume, and a systemic vascular resistance derived from the first arterial pressure waveform representative of the radial arterial pressure waveform and each of a systolic rise area and an exponential decay constant of the systolic phase derived from the second arterial waveform representative of the brachial arterial pressure waveform.

A further example of any of the foregoing methods, wherein the plurality of features can include at least one of mean arterial pressure, maximum arterial pressure, minimum arterial pressure, pulse rate, pulse pressure, maximum rate of pressure change with respect to time, minimum rate of pressure change with respect to time, end diastolic pressure, diastolic gradient, systolic pressure gradient, pulse transit time, cardiac output, stroke volume, blood temperature, systolic rise area, diastolic average pressure, systemic vascular resistance, and exponential constant associated with systolic decay.

A further example of any of the foregoing methods, wherein at least one feature of the plurality of features can be extracted from a finger arterial pressure waveform, a radial arterial pressure waveform, or a brachial arterial pressure waveform.

A further example of any of the foregoing methods, wherein the distortion score can equal a value within a nominal range of distortion scores subdivided into at least two subranges, and wherein the predetermined distortion criterion is based on a value of the distortion score associated with a threshold between subranges.

A further example of any of the foregoing methods, wherein obtaining digital hemodynamic data can include receiving a signal indicative of an arterial pressure waveform from a finger cuff.

A further example of any of the foregoing methods, wherein obtaining digital hemodynamic data can include controlling air pressure within a bladder of the finger cuff based on a blood pressure of the patient.

A further example of any of the foregoing methods, wherein the plurality of features can be extracted from a time interval starting with the application of the hemodynamic sensor to the patient or a physiological change of the patient.

A further example of any of the foregoing methods, wherein the time interval can be greater than or equal to twenty seconds and less than or equal to sixty seconds.

### A computer-readable non-transitory medium

A computer-readable non-transitory medium according to an example of this disclosure includes, among other possible things, instructions stored thereon, which when executed by a hardware processor, initiate a method. The method includes obtaining a digital hemodynamic data converted, by an analog-to-digital converter (ADC), from a signal received from a hemodynamic sensor on an ongoing basis and obtaining a first arterial pressure waveform based on the digital hemodynamic data. The method includes extracting a plurality of features from the first arterial pressure waveform, each feature indicative of distortion of the first arterial pressure waveform associated with a fit of the hemodynamic sensor to a patient. The method includes determining a distortion score corresponding to the likelihood that the hemodynamic sensor is misfit to the patient based on a weighted combination of the plurality of features and invoking a sensory alarm if the distortion score satisfies a predetermined distortion criterion.

The computer-readable non-transitory medium of the preceding paragraph can optionally include, additionally and/or alternatively, any one or more of the following features, configurations, additional components, and/or instruction steps.

A further example of the foregoing computer-readable non-transitory medium, wherein the predetermined distortion criterion can be based on at least one of a value of the distortions score and a trend of the distortion score over a time interval.

A further example of any of the foregoing computer-readable non-transitory mediums, wherein the distortion score can equal a value within a nominal range of distortion scores subdivided into at least two subranges, and wherein the predetermined distortion criterion can be based on a value of the distortion score associated with a threshold between subranges.

A further example of any of the foregoing computer-readable non-transitory mediums, wherein obtaining the first arterial pressure waveform can include transforming the digital hemodynamic data such that the first arterial pressure waveform can be representative of one of a radial arterial pressure waveform and a brachial arterial pressure waveform.

A further example of any of the foregoing computer-readable non-transitory mediums, wherein the plurality of features can include at least one of an average diastolic pressure, a stroke volume, and a systemic vascular resistance derived from the first arterial pressure waveform representative of the radial arterial pressure waveform.

A further example of any of the foregoing computer-readable non-transitory mediums can include instructions, that when executed by the hardware processor, can obtain a second arterial pressure waveform based on the digital hemodynamic data.

A further example of any of the foregoing computer-readable non-transitory mediums, wherein obtaining the second arterial pressure waveform can include transforming the digital hemodynamic data such that the second arterial pressure waveform can be representative of the other of the radial arterial pressure waveform and the brachial arterial pressure waveform.

A further example of any of the foregoing computer-readable non-transitory mediums, wherein the plurality of features can include at least one of a systolic rise area and an exponential decay constant of the systolic phase derived from the second arterial pressure waveform representative of the brachial arterial waveform.

A further example of any of the foregoing computer-readable non-transitory mediums, wherein the plurality of features can include each of an average diastolic pressure, a stroke volume, and a systemic vascular resistance derived from the first arterial pressure waveform representative of the radial arterial pressure waveform and each of a systolic rise area and an exponential decay constant of the systolic phase derived from the brachial arterial waveform.

A further example of any of the foregoing computer-readable non-transitory mediums, wherein the plurality of features can include at least one of mean arterial pressure, maximum arterial pressure, minimum arterial pressure, pulse rate, pulse pressure, maximum rate of pressure change with respect to time, minimum rate of pressure change with respect to time, end diastolic pressure, diastolic gradient, systolic pressure gradient, pulse transit time, cardiac output, stroke volume, blood temperature, systolic rise area, diastolic average pressure, systemic vascular resistance, and exponential constant associated with systolic decay.

A further example of any of the foregoing computer-readable non-transitory mediums, wherein the first arterial waveform can be representative of a finger arterial pressure waveform, a radial arterial pressure waveform, or a brachial arterial pressure waveform.

A further example of any of the foregoing computer-readable non-transitory mediums, wherein the second arterial waveform can be representative of a finger arterial pressure waveform, a radial arterial pressure waveform, or a brachial arterial pressure waveform.

A further example of any of the foregoing computer-readable non-transitory mediums, wherein the plurality of features can be extracted from a time interval starting with the application of the hemodynamic sensor to the patient or a physiological change of the patient.

A further example of any of the foregoing computer-readable non-transitory mediums, wherein the time interval can be greater than or equal to twenty seconds and less than or equal to sixty seconds.

It is intended that the invention not be limited to the particular example(s) disclosed, but that the invention will include all examples falling within the scope of the appended claims.

## Claims

1. A system (10) for monitoring hemodynamic data of a patient, the system comprising:
a hardware unit (16) including a hardware processor (24), an analog-to-digital converter (ADC) (27), and a system memory (26);
a signal distortion detection code (28) stored in the system memory and including a weighting module (30);
a hemodynamic sensor (12) coupled to the hardware unit and fitted about an appendage of the patient (14); and
a sensory alarm (37);
wherein the hardware processor is configured to execute the signal distortion detection code to:
obtain digital hemodynamic data converted, by the ADC, from a signal received from the hemodynamic sensor on an ongoing basis;
obtain (102) a first arterial pressure waveform based on the digital hemodynamic data;
extract (104) a plurality of features from the first arterial pressure waveform, wherein the plurality of features is indicative of a fit of the hemodynamic sensor to the patient;
determine (106), using the weighting module, a distortion score (34) corresponding to the likelihood that the hemodynamic sensor is misfit to the patient based on a weighted combination of the plurality of features; and
invoke (110) the sensory alarm if the distortion score satisfies a predetermined distortion criterion.

2. The system of claim 1, wherein the hemodynamic sensor is a finger cuff (42) comprising:
an inflatable bladder (50) configured to wrap around a finger of the patient;
a light-emitting diode (52); and
a photodiode (54) spaced along a longitudinal dimension of the inflatable bladder from the light-emitting diode;
wherein the first arterial pressure waveform is representative of an arterial pressure within the finger of the patient.

3. The system of claim 2, further comprising:
a pressure control unit (44) coupled to the finger cuff by a tube to receive the signal, wherein the signal is representative of an air pressure within the inflatable bladder.

4. The system of claim 3, wherein the pressure control unit comprises a control valve and a pump disposed along a conduit connecting the tube to an intake port in communication with an ambient environment, and wherein the control valve is operable to vary the air pressure within the inflatable bladder based on a blood pressure of the patient.

5. The system of claim 3, further comprising:
a patient monitoring device (18), wherein the hardware unit is housed within the pressure control unit or attached to the pressure control unit.

6. The system of any preceding claim, wherein obtaining the first arterial pressure waveform includes:
transforming (112) the digital hemodynamic data such that the first arterial pressure waveform is representative of one of a radial arterial pressure waveform and a brachial arterial pressure waveform.

7. The system of claim 6, wherein the plurality of features comprises at least one of an average diastolic pressure, a stroke volume, and a systemic vascular resistance derived from the first arterial pressure waveform representative of the radial arterial pressure waveform.

8. The system of claim 7, wherein the hardware processor is configured to execute the signal distortion detection code to:
obtain a second arterial pressure waveform based on the digital hemodynamic data, wherein obtaining the second arterial pressure waveform includes:
transforming the digital hemodynamic data such that the second arterial pressure waveform is representative of the other of the radial arterial pressure waveform and the brachial arterial pressure waveform.

9. The system of claim 8, wherein the plurality of features comprises at least one of a systolic rise area and an exponential decay constant of a systolic phase, each feature derived from the brachial arterial waveform, and
optionally wherein the plurality of features includes each of an average diastolic pressure, a stroke volume, and a systemic vascular resistance derived from the first arterial pressure waveform representative of the radial arterial pressure waveform and each of a systolic rise area and an exponential decay constant of a systolic phase derived from the second arterial pressure waveform representative of the brachial arterial pressure waveform.

10. The system of any preceding claim, wherein the plurality of features includes at least one of mean arterial pressure, maximum arterial pressure (64B), minimum arterial pressure (64A), pulse rate, pulse pressure, maximum rate of pressure change with respect to time (76E), minimum rate of pressure change with respect to time, end diastolic pressure (74A), diastolic gradient, systolic pressure gradient, pulse transit time, cardiac output, stroke volume, blood temperature, systolic rise area, diastolic average pressure, systemic vascular resistance, and exponential constant (64L) associated with systolic decay, and
optionally wherein at least one feature of the plurality of features is extracted from a finger arterial pressure waveform, a radial arterial pressure waveform, or a brachial arterial pressure waveform.

11. The system of any preceding claim, wherein the predetermined distortion criterion is based on at least one of a value of the distortion score and a trend of the distortion score over a time interval; and/or
wherein the distortion score equals a value within a nominal range of distortion scores subdivided into at least two subranges, and wherein the value of the distortion score is associated with a threshold between subranges.

12. The system of any preceding claim, wherein extracting the plurality of features from the first arterial pressure waveform includes extracting the plurality of features from a time interval starting with application of the hemodynamic sensor to the patient or physiological change of the patient,
optionally wherein the time interval is greater than or equal to twenty seconds and less than or equal to sixty seconds.

13. A method (100) for use by a system (10) for monitoring hemodynamic data of a patient, the system comprising a hemodynamic sensor (12), a sensory alarm (37), and a hardware unit (16) including a hardware processor (24), an analog-to-digital converter (ADC) (27), and a signal distortion detection code (28) stored in a system memory (26) and including a weighting module (30), the method comprising:
obtaining, by the signal distortion detection code executed by the hardware processor, digital hemodynamic data converted, by the ADC, from a signal received from the hemodynamic sensor on an ongoing basis;
obtaining (102), by the signal distortion detection code executed by the hardware processor, a first arterial pressure waveform based on the digital hemodynamic data;
extracting (104), by the signal distortion detection code executed by the hardware processor, a plurality of features from the first arterial pressure waveform indicative of distortion of the first arterial pressure waveform associated with a fit of the hemodynamic sensor to the patient;
determining (106), by the signal distortion detection code using the weighting module and executed by the hardware processor, a distortion score corresponding to the likelihood that the hemodynamic sensor is misfit to the patient based on a weighted combination of the plurality of features; and
invoking (110), by the signal distortion detection code executed by the hardware processor, the sensory alarm if the distortion score satisfies a predetermined distortion criterion.

14. The method of claim 13,
wherein the predetermined distortion criterion is based on at least one of a value of the distortion score and a trend of the distortion score over a time interval; and
wherein obtaining the first arterial pressure waveform includes:
transforming, by the signal distortion detection code executed by the hardware processor, the digital hemodynamic data such that the first arterial pressure waveform is representative of one of a radial arterial pressure waveform and a brachial arterial pressure waveform; and optionally wherein the plurality of features comprises at least one of an average diastolic pressure, a stroke volume, and a systemic vascular resistance, each feature derived from the first arterial pressure waveform representative of the radial arterial pressure waveform.

15. A computer-readable non-transitory medium having stored thereon instructions, which when executed by a hardware processor, initiate a method comprising:
obtaining a digital hemodynamic data converted, by an analog-to-digital converter (ADC), from a signal received from a hemodynamic sensor on an ongoing basis;
obtaining a first arterial pressure waveform based on the digital hemodynamic data;
extracting a plurality of features from the first arterial pressure waveform, each feature indicative of distortion of the first arterial pressure waveform associated with a fit of the hemodynamic sensor to a patient;
determining a distortion score corresponding to the likelihood that the hemodynamic sensor is misfit to the patient based on a weighted combination of the plurality of features; and
invoking a sensory alarm if the distortion score satisfies a predetermined distortion criterion.

## Patentansprüche

1. System (10) zum Überwachen hämodynamischer Daten eines Patienten, das System umfassend:
eine Hardwareeinheit (16), die einen Hardwareprozessor (24), einen Analog-Digital-Wandler (ADW) (27) und einen Systemspeicher (26) beinhaltet;
einen Programmcode zur Signalverzerrungserkennung (28), der in dem Systemspeicher gespeichert ist und ein Gewichtungsmodul (30) beinhaltet;
einen hämodynamischen Sensor (12), der mit der Hardwareeinheit gekoppelt ist und um eine Extremität des Patienten (14) herum angebracht ist; und
einen sensorischen Alarm (37);
wobei der Hardwareprozessor dazu ausgelegt ist, den Signalverzerrungsdetektionscode auszuführen, zum:
Erhalten digitaler hämodynamischer Daten, die durch den ADW aus einem Signal, das von dem hämodynamischen Sensor empfangen wird, kontinuierlich umgewandelt werden;
Erhalten (102) einer ersten arteriellen Druckkurve basierend auf den digitalen hämodynamischen Daten;
Extrahieren (104) einer Vielzahl von Merkmalen aus der ersten arteriellen Druckkurve, wobei die Vielzahl von Merkmalen eine Anpassung des hämodynamischen Sensors an den Patienten angibt;
Bestimmen (106), unter Verwendung des Gewichtungsmoduls, einer Verzerrungsbewertung (34), die der Wahrscheinlichkeit entspricht, dass der hämodynamische Sensor für den Patienten ungeeignet ist, basierend auf einer gewichteten Kombination der Vielzahl von Merkmalen; und
Aufrufen (110) des sensorischen Alarms, wenn die Verzerrungsbewertung ein vorbestimmtes Verzerrungskriterium erfüllt.

2. System nach Anspruch 1, wobei der hämodynamische Sensor eine Fingermanschette (42) ist, umfassend:
eine aufblasbare Blase (50), dazu ausgelegt, sich um einen Finger des Patienten zu wickeln;
eine Leuchtdiode (52); und
eine Fotodiode (54), die entlang einer Längsabmessung der aufblasbaren Blase von der Leuchtdiode beabstandet ist;
wobei die erste arterielle Druckkurve einen arteriellen Druck innerhalb des Fingers des Patienten darstellt.

3. System nach Anspruch 2, ferner umfassend:
eine Drucksteuereinheit (44), die mit der Fingermanschette durch einen Schlauch gekoppelt ist, um das Signal zu empfangen, wobei das Signal einen Luftdruck innerhalb der aufblasbaren Blase darstellt.

4. System nach Anspruch 3, wobei die Drucksteuereinheit ein Steuerventil und eine Pumpe umfasst, die entlang einer Leitung angeordnet sind, die den Schlauch mit einem Einlassanschluss in Kommunikation mit einer Umgebungsumgebung verbindet, und wobei das Steuerventil dazu betreibbar ist, den Luftdruck innerhalb der aufblasbaren Blase basierend auf einem Blutdruck des Patienten zu variieren.

5. System nach Anspruch 3, ferner umfassend:
eine Patientenüberwachungsvorrichtung (18), wobei die Hardwareeinheit innerhalb der Drucksteuereinheit untergebracht oder an der Drucksteuereinheit befestigt ist.

6. System nach einem der vorhergehenden Ansprüche, wobei das Erhalten der ersten arteriellen Druckkurve beinhaltet:
Transformieren (112) der digitalen hämodynamischen Daten, sodass die erste arterielle Druckkurve eines aus einer radialarteriellen Druckkurve und einer brachialarteriellen Druckkurve darstellt.

7. System nach Anspruch 6, wobei die Vielzahl von Merkmalen einen durchschnittlichen diastolischen Druck und/oder ein Schlagvolumen und/oder einen systemischen Gefäßwiderstand umfasst, der von der ersten arteriellen Druckkurve abgeleitet ist, die die radialarterielle Druckkurve darstellt.

8. System nach Anspruch 7, wobei der Hardwareprozessor dazu ausgelegt ist, den Programmcode zur Signalverzerrungserkennung auszuführen, um:
Erhalten einer zweiten arteriellen Druckkurve basierend auf den digitalen hämodynamischen Daten, wobei das Erhalten der zweiten arteriellen Druckkurve beinhaltet: Transformieren der digitalen hämodynamischen Daten, sodass die zweite arterielle Druckkurve das andere aus der radialarteriellen Druckkurve und der brachialarteriellen Druckkurve darstellt.

9. System nach Anspruch 8, wobei die Vielzahl von Merkmalen einen systolischen Anstiegsbereich und/oder eine exponentielle Druckabfallkonstante einer systolischen Phase umfasst, wobei jedes Merkmal von der brachialarteriellen Druckkurve abgeleitet ist, und optional wobei die Vielzahl von Merkmalen jeweils einen durchschnittlichen diastolischen Druck, ein Schlagvolumen und einen systemischen Gefäßwiderstand beinhaltet, der von der ersten arteriellen Druckkurve abgeleitet ist, die die radialarterielle Druckkurve darstellt, und jeweils einen systolischen Anstiegsbereich und eine exponentielle Druckabfallkonstante einer systolischen Phase, die von der zweiten arteriellen Druckkurve abgeleitet ist, die die brachialarterielle Druckkurve darstellt.

10. System nach einem der vorhergehenden Ansprüche, wobei die Vielzahl von Merkmalen mindestens eines von mittlerem arteriellen Druck, maximalem arteriellen Druck (64B), minimalem arteriellen Druck (64A), Pulsrate, Pulsdruck, maximaler Druckänderungsrate in Bezug auf die Zeit (76E), minimaler Druckänderungsrate in Bezug auf die Zeit, enddiastolischem Druck (74A), diastolischem Gradienten, systolischem Druckgradienten, Pulsdurchgangszeit, Herzzeitvolumen, Schlagvolumen, Bluttemperatur, systolischer Anstiegsfläche, diastolischem Durchschnittsdruck, systemischem Gefäßwiderstand, und Exponentialkonstante (64L), in Verbindung mit systolischem Druckabfall beinhaltet, und optional wobei mindestens ein Merkmal der Vielzahl von Merkmalen aus einer arteriellen Fingerdruckkurve, einer radialarteriellen Druckkurve oder einer brachialarteriellen Druckkurve extrahiert wird.

11. System nach einem der vorhergehenden Ansprüche, wobei das vorbestimmte Verzerrungskriterium auf einem Wert der Verzerrungsbewertung und/oder einem Trend der Verzerrungsbewertung über ein Zeitintervall basiert; und/oder
wobei die Verzerrungsbewertung gleich einem Wert innerhalb eines nominalen Bereichs von Verzerrungsbewertungen ist, die in mindestens zwei Teilbereiche unterteilt sind, und wobei der Wert der Verzerrungsbewertung mit einer Schwelle zwischen Teilbereichen verknüpft ist.

12. System nach einem der vorhergehenden Ansprüche, wobei das Extrahieren der Vielzahl von Merkmalen aus der ersten arteriellen Druckkurve das Extrahieren der Vielzahl von Merkmalen aus einem Zeitintervall, das mit der Anwendung des hämodynamischen Sensors auf den Patienten oder der physiologischen Änderung des Patienten beginnt, beinhaltet,
wobei optional das Zeitintervall mindestens zwanzig Sekunden und höchstens sechzig Sekunden lang ist.

13. Verfahren (100) zur Verwendung durch ein System (10) zum Überwachen hämodynamischer Daten eines Patienten, das System umfassend einen hämodynamischen Sensor (12), einen sensorischen Alarm (37) und eine Hardwareeinheit (16), die einen Hardwareprozessor (24), einen Analog-Digital-Wandler (ADW) (27) und einen Programmcode zur Signalverzerrungserkennung (28) beinhaltet, der in einem Systemspeicher (26) gespeichert ist und ein Gewichtungsmodul (30) umfasst, das Verfahren umfassend:
Erhalten, durch den Programmcode zur Signalverzerrungserkennung, der durch den Hardwareprozessor ausgeführt wird, digitaler hämodynamischer Daten, die durch den ADW von einem Signal umgewandelt werden, das kontinuierlich von dem hämodynamischen Sensor empfangen wird;
Erhalten (102), durch den Signalverzerrungsdetektionscode, der durch den Hardwareprozessor ausgeführt wird, einer ersten arteriellen Druckkurve basierend auf den digitalen hämodynamischen Daten;
Extrahieren (104), durch den Signalverzerrungsdetektionscode, der durch den Hardwareprozessor ausgeführt wird, einer Vielzahl von Merkmalen aus der ersten arteriellen Druckkurve, die eine Verzerrung der ersten arteriellen Druckkurve angibt, die mit einer Anpassung des hämodynamischen Sensors an den Patienten verknüpft ist;
Bestimmen (106), durch den Programmcode zur Signalverzerrungserkennung unter Verwendung des Gewichtungsmoduls und ausgeführt durch den Hardwareprozessor, einer Verzerrungsbewertung, die der Wahrscheinlichkeit entspricht, dass der hämodynamische Sensor für den Patienten ungeeignet ist, basierend auf einer gewichteten Kombination der Vielzahl von Merkmalen; und
Aufrufen (110), durch den Programmcode zur Signalverzerrungserkennung, der durch den Hardwareprozessor ausgeführt wird, des sensorischen Alarms, falls die Verzerrungsbewertung ein vorbestimmtes Verzerrungskriterium erfüllt.

14. Verfahren nach Anspruch 13,
wobei das vorbestimmte Verzerrungskriterium auf einem Wert der Verzerrungsbewertung und/oder einem Trend der Verzerrungsbewertung über ein Zeitintervall basiert; und wobei das Erhalten der ersten arteriellen Druckkurve beinhaltet:
Transformieren, durch den Signalverzerrungsdetektionscode, der durch den Hardwareprozessor ausgeführt wird, der digitalen hämodynamischen Daten, sodass die erste arterielle Druckkurve eine von einer radialarteriellen Druckkurve und einer brachialarteriellen Druckkurve darstellt; und
optional wobei die Vielzahl von Merkmalen mindestens eines von einem durchschnittlichen diastolischen Druck, einem Schlagvolumen und einem systemischen Gefäßwiderstand umfasst, wobei jedes Merkmal von der ersten arteriellen Druckkurve abgeleitet ist, die repräsentativ für die arterielle Druckkurve ist.

15. Computerlesbares, nichtflüchtiges Medium mit darauf gespeicherten Anweisungen, die, wenn durch einen Hardwareprozessor ausgeführt, ein Verfahren einleiten, umfassend:
Erhalten digitaler hämodynamischer Daten, die durch einen Analog-Digital-Wandler (ADC) aus einem Signal, das von einem hämodynamischen Sensor empfangen wird, kontinuierlich umgewandelt werden;
Erhalten einer ersten arteriellen Druckkurve basierend auf den digitalen hämodynamischen Daten;
Extrahieren einer Vielzahl von Merkmalen aus der ersten arteriellen Druckkurve, wobei jedes Merkmal eine Verzerrung der ersten arteriellen Druckkurve angibt, die mit einer Anpassung des hämodynamischen Sensors an einen Patienten verknüpft ist;
Bestimmen einer Verzerrungsbewertung, die der Wahrscheinlichkeit entspricht, dass der hämodynamische Sensor für den Patienten ungeeignet ist, basierend auf einer gewichteten Kombination der Vielzahl von Merkmalen; und
Aufrufen eines sensorischen Alarms, wenn die Verzerrungsbewertung ein vorbestimmtes Verzerrungskriterium erfüllt.

## Revendications

1. Système (10) permettant de surveiller des données hémodynamiques d'un patient, le système comprenant :
une unité matérielle (16) comportant un processeur matériel (24), un convertisseur analogique-numérique (CAN) (27), et une mémoire système (26) ;
un code (28) de détection de distorsion de signal stocké dans la mémoire système et comportant un module de pondération (30) ;
un capteur hémodynamique (12) couplé à l'unité matérielle et monté autour d'un appendice du patient (14) ; et
une alarme sensorielle (37) ;
dans lequel le processeur matériel est configuré pour exécuter le code de détection de distorsion de signal pour :
obtenir des données hémodynamiques numériques converties, par le CAN, à partir d'un signal reçu du capteur hémodynamique en continu ;
obtenir (102) une première forme d'onde de pression artérielle basée sur les données hémodynamiques numériques ;
extraire (104) une pluralité de caractéristiques de la première forme d'onde de pression artérielle, la pluralité de caractéristiques indiquant un ajustement du capteur hémodynamique au patient ;
déterminer (106), à l'aide du module de pondération, un score de distorsion (34) correspondant à la probabilité que le capteur hémodynamique ne soit pas adapté au patient sur la base d'une combinaison pondérée de la pluralité de caractéristiques ; et
invoquer (110) l'alarme sensorielle si le score de distorsion satisfait à un critère de distorsion prédéterminé.

2. Système selon la revendication 1, dans lequel le capteur hémodynamique est un manchon de doigt (42) comprenant :
une vessie gonflable (50) configurée pour s'enrouler autour d'un doigt du patient ;
une diode électroluminescente (52) ; et
une photodiode (54) espacée de la diode électroluminescente le long d'une dimension longitudinale de la vessie gonflable ;
dans lequel la première forme d'onde de pression artérielle est représentative d'une pression artérielle au sein du doigt du patient.

3. Système selon la revendication 2, comprenant en outre :
une unité de commande de pression (44) couplée au manchon de doigt par un tube pour recevoir le signal, le signal étant représentatif d'une pression d'air à l'intérieur de la vessie gonflable.

4. Système selon la revendication 3, dans lequel l'unité de commande de pression comprend une soupape de commande et une pompe disposée le long d'un conduit reliant le tube à un orifice d'admission en communication avec un environnement ambiant, et dans lequel la soupape de commande peut être actionnée pour faire varier la pression d'air à l'intérieur de la vessie gonflable sur la base d'une pression artérielle du patient.

5. Système selon la revendication 3, comprenant en outre :
un dispositif de surveillance de patient (18), dans lequel l'unité matérielle est logée à l'intérieur de l'unité de commande de pression ou fixée à l'unité de commande de pression.

6. Système selon une quelconque revendication précédente, dans lequel l'obtention de la première forme d'onde de pression artérielle comporte :
la transformation (112) des données hémodynamiques numériques de telle sorte que la première forme d'onde de pression artérielle soit représentative d'une forme d'onde de pression artérielle radiale et d'une forme d'onde de pression artérielle brachiale.

7. Système selon la revendication 6, dans lequel la pluralité de caractéristiques comprend au moins l'un d'une pression diastolique moyenne, d'un volume systolique et d'une résistance vasculaire systémique dérivée de la première forme d'onde de pression artérielle représentative de la forme d'onde de pression artérielle radiale.

8. Système selon la revendication 7, dans lequel le processeur matériel est configuré pour exécuter le code de détection de distorsion de signal pour :
obtenir une seconde forme d'onde de pression artérielle sur la base des données hémodynamiques numériques, l'obtention de la seconde forme d'onde de pression artérielle comportant :
la transformation des données hémodynamiques numériques de telle sorte que la seconde forme d'onde de pression artérielle soit représentative de l'autre de la forme d'onde de pression artérielle radiale et de la forme d'onde de pression artérielle brachiale.

9. Système selon la revendication 8, dans lequel la pluralité de caractéristiques comprend au moins l'une d'une aire de montée systolique et d'une constante de décroissance exponentielle d'une phase systolique, chaque caractéristique étant dérivée de la forme d'onde artérielle brachiale, et
éventuellement dans lequel la pluralité de caractéristiques comporte chacun d'une pression diastolique moyenne, d'un volume systolique et d'une résistance vasculaire systémique dérivée de la première forme d'onde de pression artérielle représentative de la forme d'onde de pression artérielle radiale et chacune d'une aire de montée systolique et d'une constante de décroissance exponentielle d'une phase systolique dérivée de la seconde forme d'onde de pression artérielle représentative de la forme d'onde de pression artérielle brachiale.

10. Système selon une quelconque revendication précédente, dans lequel la pluralité de caractéristiques comporte au moins l'une parmi la pression artérielle moyenne, la pression artérielle maximale (64B), la pression artérielle minimale (64A), la fréquence de pouls, la pression de pouls, le taux maximal de variation de la pression par rapport au temps (76E), le taux minimal de variation de la pression par rapport au temps, la pression en fin de diastole (74A), le gradient diastolique, le gradient de pression systolique, le temps de transit du pouls, le débit cardiaque, le volume systolique, la température sanguine, l'aire de montée systolique, la pression moyenne diastolique, la résistance vasculaire systémique, et la constante exponentielle (64L) associée à la décroissance systolique, et
éventuellement dans lequel au moins une caractéristique de la pluralité de caractéristiques est extraite d'une forme d'onde de pression artérielle du doigt, d'une forme d'onde de pression artérielle radiale ou d'une forme d'onde de pression artérielle brachiale.

11. Système selon une quelconque revendication précédente, dans lequel le critère de distorsion prédéterminé est basé sur au moins l'une d'une valeur du score de distorsion et d'une tendance du score de distorsion sur un intervalle de temps ; et/ou
dans lequel le score de distorsion est égal à une valeur à l'intérieur d'une plage nominale de scores de distorsion subdivisée en au moins deux sous-plages, et dans lequel la valeur du score de distorsion est associée à un seuil entre des sous-plages.

12. Système selon une quelconque revendication précédente, dans lequel l'extraction de la pluralité de caractéristiques à partir de la première forme d'onde de pression artérielle comporte l'extraction de la pluralité de caractéristiques à partir d'un intervalle de temps commençant par l'application du capteur hémodynamique au patient ou un changement physiologique du patient. éventuellement dans lequel l'intervalle de temps est supérieur ou égal à vingt secondes et inférieur ou égal à soixante secondes.

13. Procédé (100) destiné à être utilisé par un système (10) de surveillance de données hémodynamiques d'un patient, le système comprenant un capteur hémodynamique (12), une alarme sensorielle (37), et une unité matérielle (16) comportant un processeur matériel (24), un convertisseur analogique-numérique (CAN) (27), et un code de détection de distorsion de signal (28) stocké dans une mémoire système (26) et comportant un module de pondération (30), le procédé comprenant :
l'obtention, par le code détection de distorsion de signal exécuté par le processeur matériel, de données hémodynamiques numériques converties, par le CAN, à partir d'un signal reçu du capteur hémodynamique en continu ;
l'obtention (102), par le code de détection de distorsion de signal exécuté par le processeur matériel, d'une première forme d'onde de pression artérielle sur la base des données hémodynamiques numériques ;
l'extraction (104), par le code de détection de distorsion de signal exécuté par le processeur matériel, d'une pluralité de caractéristiques à partir de la première forme d'onde de pression artérielle révélatrices d'une distorsion de la première forme d'onde de pression artérielle associée à un ajustement du capteur hémodynamique au patient ;
la détermination (106), par le code de détection de distorsion de signal au moyen du module de pondération et exécuté par le processeur matériel, d'un score de distorsion correspondant à la probabilité que le capteur hémodynamique ne soit pas adapté au patient sur la base d'une combinaison pondérée de la pluralité de caractéristiques ; et
l'invocation (110), par le code de détection de distorsion de signal exécuté par le processeur matériel, de l'alarme sensorielle si le score de distorsion satisfait à un critère de distorsion prédéterminé.

14. Procédé selon la revendication 13,
dans lequel le critère de distorsion prédéterminé est basé sur au moins l'une d'une valeur du score de distorsion et d'une tendance du score de distorsion sur un intervalle de temps ; et
dans lequel l'obtention de la première forme d'onde de pression artérielle comporte :
la transformation, par le code de détection de distorsion de signal exécuté par le processeur matériel, des données hémodynamiques numériques de telle sorte que la première forme d'onde de pression artérielle soit représentative d'une parmi une forme d'onde de pression artérielle radiale et une forme d'onde de pression artérielle brachiale ; et éventuellement dans lequel la pluralité de caractéristiques comprend au moins l'un d'une pression diastolique moyenne, d'un volume systolique et d'une résistance vasculaire systémique, chaque caractéristique dérivée de la première forme d'onde de pression artérielle étant représentative de la forme d'onde de pression artérielle radiale.

15. Support non transitoire lisible par ordinateur sur lequel sont stockées des instructions qui, lorsqu'elles sont exécutées par un processeur matériel, lancent un procédé comprenant :
l'obtention de données hémodynamiques numériques converties, par un convertisseur analogique-numérique (CAN), à partir d'un signal reçu du capteur hémodynamique en continu ;
l'obtention d'une première forme d'onde de pression artérielle basée sur les données hémodynamiques numériques ;
l'extraction d'une pluralité de caractéristiques de la première forme d'onde de pression artérielle, chaque caractéristique étant révélatrice d'une distorsion de la première forme d'onde de pression artérielle associée à un ajustement du capteur hémodynamique à un patient ;
la détermination d'un score de distorsion correspondant à la probabilité que le capteur hémodynamique ne soit pas adapté au patient sur la base d'une combinaison pondérée de la pluralité de caractéristiques ; et
l'invocation d'une alarme sensorielle si le score de distorsion satisfait à un critère de distorsion prédéterminé.
